# EUROPEAN PATENT SPECIFICATION

(11) **EP 1 143 885 B1**
(45) Date of publication and mention of the grant of the patent: **27.02.2008**
(21) Application number: 00903218.6
(22) Date of filing: 11.01.2000
(51) Int. Cl.: A61F 2/44, A61F 2/46

(54) **TRUNCATED OPEN INTERVERTEBRAL SPACERS**
ABGESTUMPFTES, OFFENES ZWISCHENWIRBELDISTANZSTÜCK
ELEMENT D'ESPACEMENT INTERVERTEBRAUX, OUVERTS ET TRONQUES

(30) Priority: 11.01.1999 US 115388 P
(43) Date of publication of application: 17.10.2001
(73) Proprietor: SDGI Holdings, Inc., Wilmington, DE 19801 (US)
(72) Inventor: BOYD, Lawrence, M., Memphis, TN 38104 (US); BURKUS, J., Kenneth, Columbus, GA 31904 (US); DORCHAK, John, D., Midland, GA 31820 (US); ESTES, Bradley, T., Memphis, TN 38117 (US); RAY, Eddie, F.,III, Collierville, TN 38017 (US)
(74) Representative: Allman, Peter John
(86) International application number: PCT/US2000/000590
(87) International publication number: WO 2000/041654

(56) References cited:
- EP-A- 0 421 485
- WO-A-98/55052
- US-A- 4 736 738
- US-A- 5 423 825
- US-A- 5 593 409
- US-A- 5 658 337
- US-A- 6 033 438

## Description

### BACKGROUND OF THE INVENTION

The present invention broadly concerns arthrodesis for stabilizing the spine. More specifically, the invention provides open-chambered intervertebral spacers, instruments for implanting the spacers and methods for making and using the spacers.

Intervertebral discs, located between the endplates of adjacent vertebrae, stabilize the spine, distribute forces between vertebrae and cushion vertebral bodies. A normal intervertebral disc includes a semi-gelatinous component, the nucleus pulposus, which is surrounded and confined by an outer, fibrous ring called the annulus fibrosus. In a healthy, undamaged spine, the annulus fibrosus prevents the nucleus pulposus from protruding outside the disc space.

Spinal discs may be displaced or damaged due to trauma, disease or aging. Disruption of the annulus fibrosus allows the nucleus pulposus to protrude into the vertebral canal, a condition commonly referred to as a herniated or ruptured disc. The extruded nucleus pulposus may press on a spinal nerve, which may result in nerve damage, pain, numbness, muscle weakness and paralysis. Intervertebral discs may also deteriorate due to the normal aging process or disease. As a disc dehydrates and hardens, the disc space height will be reduced leading to instability of the spine, decreased mobility and pain.

Sometimes the only relief from the symptoms of these conditions is a discectomy, or surgical removal of a portion or all of an intervertebral disc followed by fusion of the adjacent vertebrae. The removal of the damaged or unhealthy disc will allow the disc space to collapse. Collapse of the disc space can cause instability of the spine, abnormal joint mechanics, premature development of arthritis or nerve damage, in addition to severe pain. Pain relief via discectomy and arthrodesis requires preservation of the disc space and eventual fusion of the affected motion segments.

Bone grafts are often used to fill the intervertebral space to prevent disc space collapse and promote fusion of the adjacent vertebrae across the disc space. In early techniques, bone material was simply disposed between the adjacent vertebrae, typically at the posterior aspect of the vertebra, and the spinal column was stabilized by way of a plate or rod spanning the affected vertebrae. Once fusion occurred, the hardware used to maintain the stability of the segment became superfluous and was a permanent foreign body. Moreover, the surgical procedures necessary to implant a rod or plate to stabilize the level during fusion were frequently lengthy and involved.

It was therefore determined that a more optimal solution to the stabilization of an excised disc space is to fuse the vertebrae between their respective end plates, preferably without the need for anterior or posterior plating. There have been an extensive number of attempts to develop an acceptable intradiscal implant that could be used to replace a damaged disc and maintain the stability of the disc interspace between the adjacent vertebrae, at least until complete arthrodesis is achieved. The implant must provide temporary support and allow bone ingrowth. Success of the discectomy and fusion procedure requires the development of a contiguous growth of bone to create a solid mass because the implant may not withstand the compressive loads on the spine for the life of the patient.

Several metal spacers have been developed to fill the void formed and to promote fusion. Sofamor Danek Group, Inc., (1800 Pyramid Place, Memphis, TN 38132, (800) 933-2635) markets a number of hollow spinal cages. For example, U.S. Patent No. 5,015,247 to Michelson and U.S. Serial No. 08/411,017 to Zdeblick disclose a threaded spinal cage. The cages are hollow and can be filled with osteogenic material, such as autograft or allograft, prior to insertion into the intervertebral space. Apertures defined in the cage communicate with the hollow interior to provide a path for tissue growth between the vertebral endplates.

Although the metal fusion devices of Sofamor Danek and others are widely and successfully employed for reliable fusions, it is sometimes desirable to use an all-bone product. Bone provides many advantages for use in fusions. It can be incorporated after fusion occurs and therefore will not be a permanent implant. Bone allows excellent postoperative imaging because it does not cause scattering like metallic implants. Stress shielding is avoided because bone grafts have a similar modulus of elasticity as the surrounding bone. Although an all-bone spacer provides these and other benefits, the use of bone presents several challenges. Any spacer which will be placed within the intervertebral disc space must withstand the cyclic loads of the spine. Cortical bone products may have sufficient compressive strength for such use, however, cortical bone will not promote rapid fusion. Cancellous bone is more conducive to fusion but is not biomechanically sound as an intervertebral spacer.

WO 98/55052 discloses an interbody fusion spacer having a body that defines a chamber and an opening in communication with the chamber. The body has a pair of spaced arm that form a mouth to the chamber. One of the arm is truncated relative to the other to form a channel that is designed to facilitate implantation with an insertion tool.

According to the present invention there is provided an interbody fusion spacer, comprising: an elongated body defining a chamber, said body having a first arm and a second opposing arm, said arms defining an opening in communication with said chamber, characterised in that each of said first arm and second arm has an end configured to form a nesting region within which an adjacent spacer can nest.

This invention provides spacers having an open chamber, tools for implanting the spacers and methods for making and using the spacers. The spacers include an elongated body defining a chamber and having a first arm and a second arm. The arms define an opening in communication with the chamber. In one preferred embodiment, the ends are substantially identical to each other. In a preferred form of the invention, the arms each have an end with a concave surface extending to the outer edge or periphery of the spacer.

Spacers of the invention desirably have bodies composed of bone. In one aspect, the body is a dowel having a substantially C-shaped chamber and comprising an off-center bone plug obtained from the diaphysis of a long bone.

Another aspect of the invention provides interbody fusion implant systems. In one form of the invention, the system includes two of the inventive spacers described above in nestable combination. In another form of the invention, the system includes one of the inventive spacers described above along with a second spacer nestable therewith. The second spacer may for example have an elongated body and an outer surface, and may define a through-hole that preferably extends perpendicular to the longitudinal axis of the second body.

Tools for implanting spacers are also provided. The tools include spacer engaging means for engaging a spacer and occlusion means for blocking an opening defined in the spacer. In one aspect the engaging means includes a shaft slidingly disposed within a housing and having a threaded post for engaging a threaded tool hole in the spacer. In some embodiments, the occlusion means includes a plate extendible from the housing. In one specific embodiment the plate defines a groove which is disposed around a fastener attached to the housing so that the plate is slideable relative to the housing. In other preferred embodiments, the occlusion member has an interior and exterior surface wherein at least one of the surfaces is curved.

This invention also includes methods for obtaining an open bone dowel and methods for using the spacers of this invention. The methods of making a dowel according to this invention include cutting an off-center plug from the diaphysis of a long bone to obtain a bone dowel having an open chamber. The dowel is machined to include desirable nestable surface features such as those described above, in addition to threads, grooves, instrument holes and the like. In still another aspect, the methods include chamfering the forward end of the dowel.

The invention also concerns methods for using the spacers of this invention to promote fusion bone growth between adjacent vertebrae. In one form of the invention, the method for promoting fusion bone growth includes providing one of the spacers described above, preparing the adjacent vertebrae to receive the elongated body of the spacer by, for example, making a cavity between two vertebrae to be fused and implanting or otherwise placing the elongated body into the intervertebral space. In some embodiments the chamber is packed with osteogenic material before the spacer is implanted. In. other aspects of the invention, osteogenic material is packed into and around the chamber through the mouth or channel after implantation.

The combination of the open-chambered spacers of this invention with the tools and methods of this invention provide a versatile spacer and implant systems. The spacers can be packed before or after implantation. This invention facilitates implanting a pair of open spacers close to each other in an intervertebral space.

Accordingly, it is one object of this invention to provide nestable bone fusion spacers and methods for using the spacers in arthrodesis procedures.

Another object of this invention is to provide a dowel for vertebral fusions which has improved properties and versatility over standard dowels known in the art.

These and other objects, advantages and features are accomplished according to the spacers, tools and methods of the following description of the preferred embodiments of the present invention.

### BRIEF DESCRIPTION OF THE DRAWINGS

Figure 1 is a perspective view of an interbody fusion spacer of the present invention.
Figure 2 is an end elevational view of the interbody fusion spacer depicted in Figure 1.
Figure 3 is a perspective view of an implant system including two C-shaped spacers having arcuately truncated arms.
Figure 4 is a top elevational view of the implant system depicted in Figure 3.
Figure 5 is an end elevational view of the implant system depicted in Figure 3.
Figure 6 is a perspective view of an implant system of the present invention, showing how the spacer of Figure 1 may be alternately positioned adjacent to another of said spacers.
Figure 7 is a top elevational view of the implant system depicted in Figure 6.
Figure 8 is an end elevational view of the implant system depicted in Figure 6.
Figure 9 is a perspective view of an implant system including one C-shaped spacer nested with another spacer.
Figure 10 is a top elevational view of the implant system depicted in Figure 9.
Figure 11 is an end elevational view of the implant system depicted in Figure 9.
Figure 12 is a top perspective view of one embodiment of an insertion tool of the invention.
Figure 13 is a side perspective view of the insertion tool depicted in Figure 12.
Figure 14 is an exploded side perspective view of a tool-spacer assembly according to this invention.
Figure 15 is a side perspective view of the tool-spacer assembly depicted in Figure 14.

### DESCRIPTION OF THE PREFERRED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

This invention provides spacers having an open-mouthed chamber. These spacers are advantageous for maximum exposure of vertebral tissue to osteogenic material within the chamber and allow close placement of a pair of spacers within the intervertebral space. The design of these spacers conserves material without compromising biomechanical properties of the spacer. This is particularly advantageous when the material is bone because the invention preserves precious allograft. In fact, larger dowels and other shaped grafts can be obtained from smaller bones than was ever thought possible before the present invention. Likewise, smaller dowels having a pre-formed chamber may be efficiently obtained from larger bones.

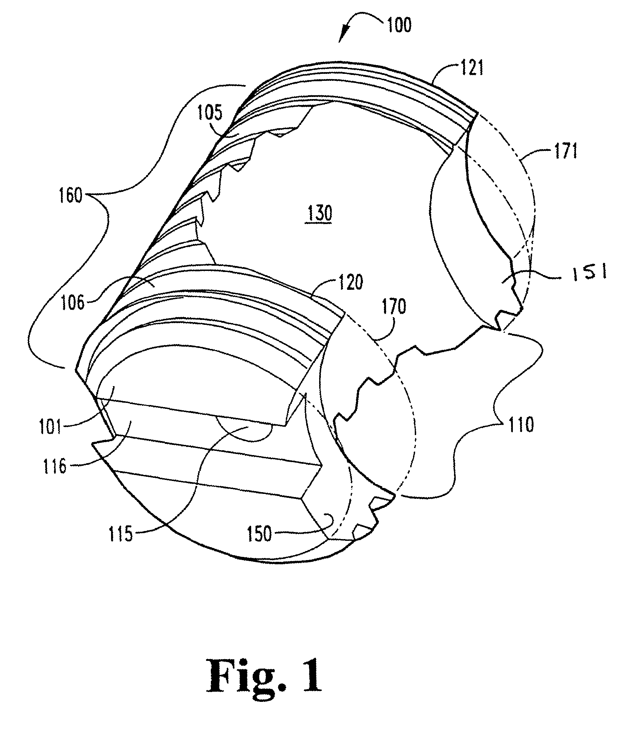

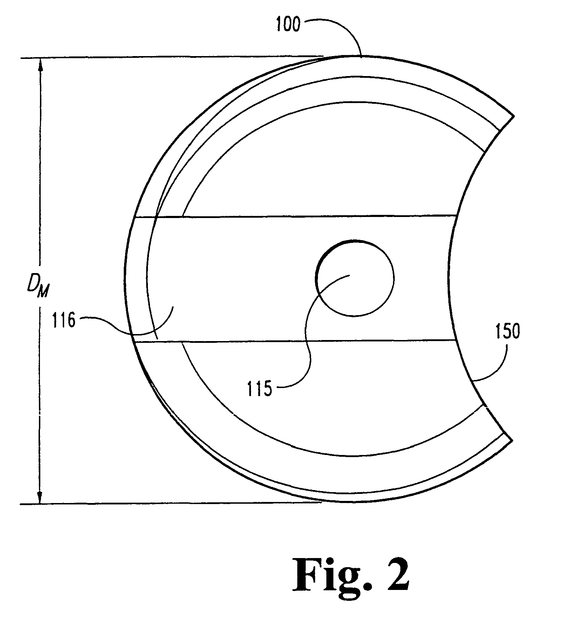

As disclosed, one aspect of the invention provides interbody fusion spacers configured to nest with an adjacent spacer. Referring now to Figures 1 and 2, in one embodiment, a generally C-shaped spacer 100 is provided. Spacer 100 includes an elongated body 105 having an outer surface 106 and a maximum outer diameter D_{M}. Elongated body 105 defines an internal, or interior, cavity 130 useful for receiving and holding graft material for promoting bone ingrowth. Body 105 further defines a first arm 120 and a second opposing arm 121 similar to the spacers described above. Arms 120, 121 define an opening, or mouth, 110 in communication with interior cavity 130. Arm 120 has an end 150 that is configured to form a region 170 within which an adjacent spacer can nest as best seen in Figure 1. Similarly, arm 121 has an end 151 that is configured to form a region 171 within which an adjacent spacer can nest. End 151 is preferably substantially identical to end 150. In one form of the invention as seen in Figure 1, ends 150 and 151 are arcuately truncated. The generally concave configuration of surfaces of ends 150 and 151 are useful, for example, in allowing spacer 100 to be positioned more closely to an adjacent spacer if desired, thus allowing the surface of the ends of the arms to receive a convex surface of an adjacent spacer, and/or allows a clearance adjacent to the spacers. Such clearance may be useful, for instance, in easing threaded (rotary) insertion of an adjacent spacer in close proximity, particularly when also using an insertion tool such as that described herein. Thus, so designed, spacer 100 is generally C-shaped, having on one side thereof a wall 160 which is continuous along its entire length, whereas on the opposed side the spacer 100 lacks such a continuous wall and rather has arms 120 and 121 extending from wall 160, with ends 150 and 151 of arms 120 and 121 exposed oppositely from said wall 160.

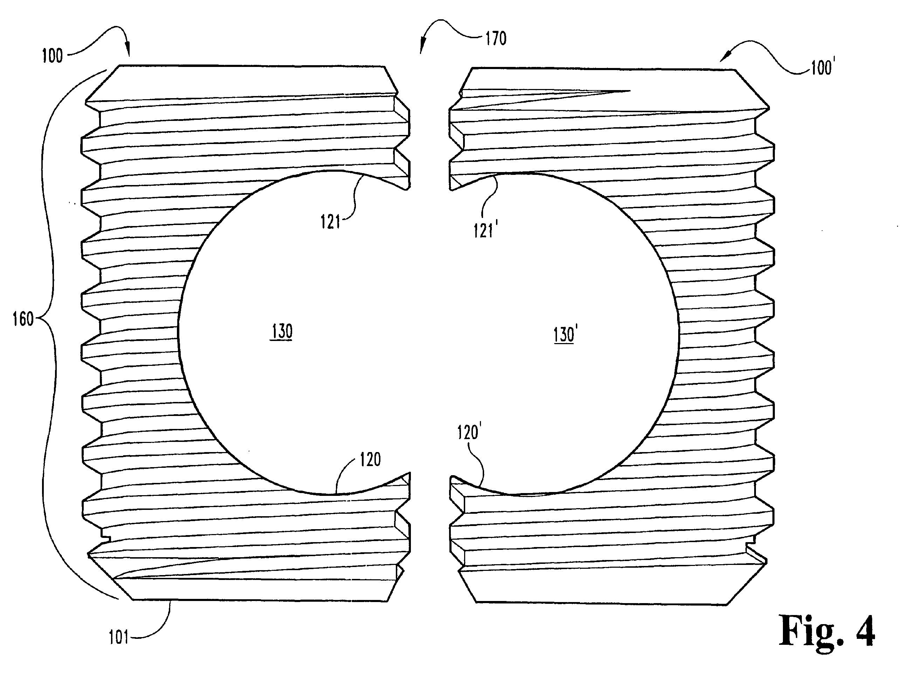

Spacer 100 can have all of the features of other generally C-shaped spacers, for example as disclosed in International Application Serial No. PCT/US98/11159 filed June 3, 1998 entitled OPEN INTERVERTEBRAL SPACERS, published as WO 98/55052 on December 10, 1998, which is hereby incorporated by reference in its entirety. For example, spacer 100 can include a first, tool-engaging end 101 having a tool engagement hole 115 and slot 116 therein as seen in FIGS. 1 and 2. Outer surface of spacer 100 can also define threaded bone-engaging portions or other surface features as described above. Threads 107 are best seen in Figure 4.

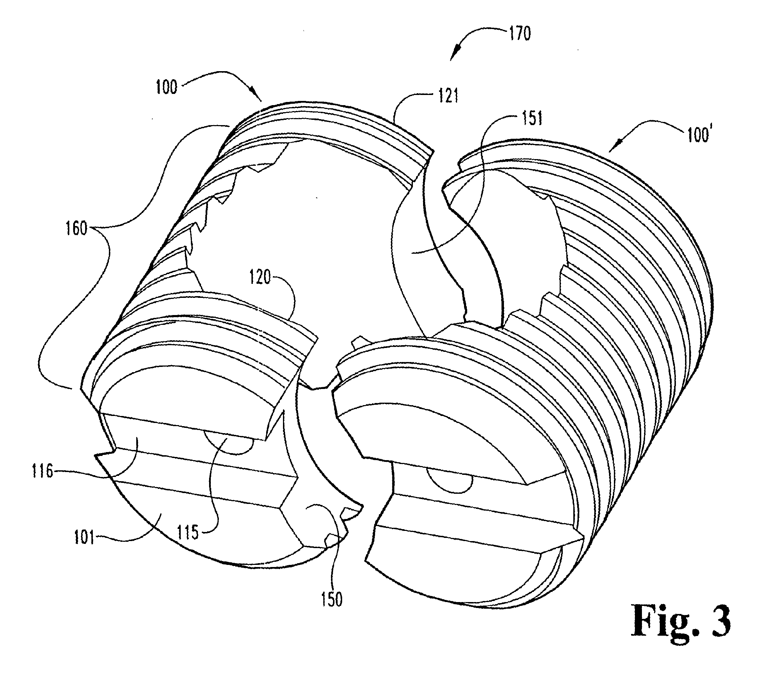

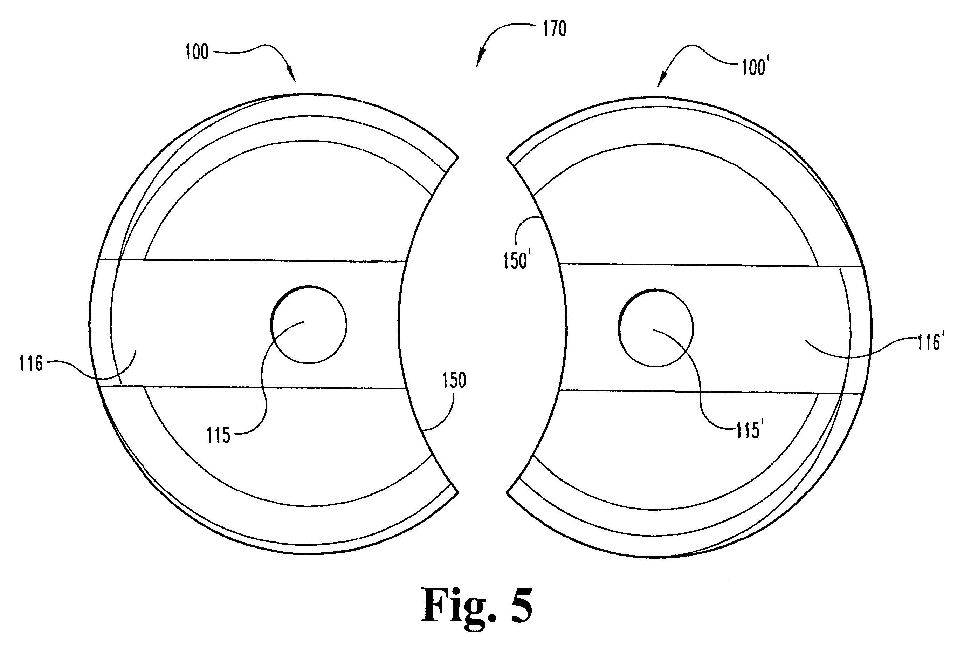

In yet another aspect of the invention, interbody fusion implant systems are provided. Referring now to FIGS. 3-5, one form of an implant system 170 includes a first, generally C-shaped spacer 100 as described above, and a second generally C-shaped spacer 100' of similar design. Therefore, features of spacer 100' are numbered correspondingly to those of spacer 100, except with a denoting prime (') symbol. In one mode of practicing the invention, spacers 100 and 100' are obtained by taking a transverse plug from a diaphysis of a long bone having a medullary canal, as discussed for example in U.S. Patent No. 5,814,084 entitled DIAPHYSIAL CORTICAL DOWEL, which is hereby incorporated herein by reference in its entirety, and/or in the above-cited International Application Serial No. PCT/US98/11159 filed June 3, 1998, and machined to have additional features as described herein. Thus, the medullary canal forms at least a portion of the cavities 130 and 130' of spacers 100 and 100', respectively. Spacers 100 and 100' are also preferably threaded.

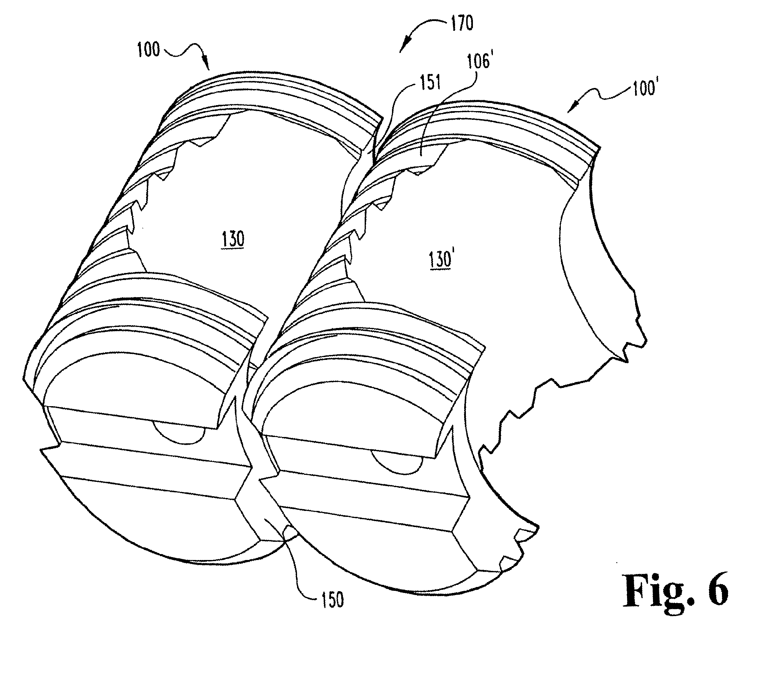

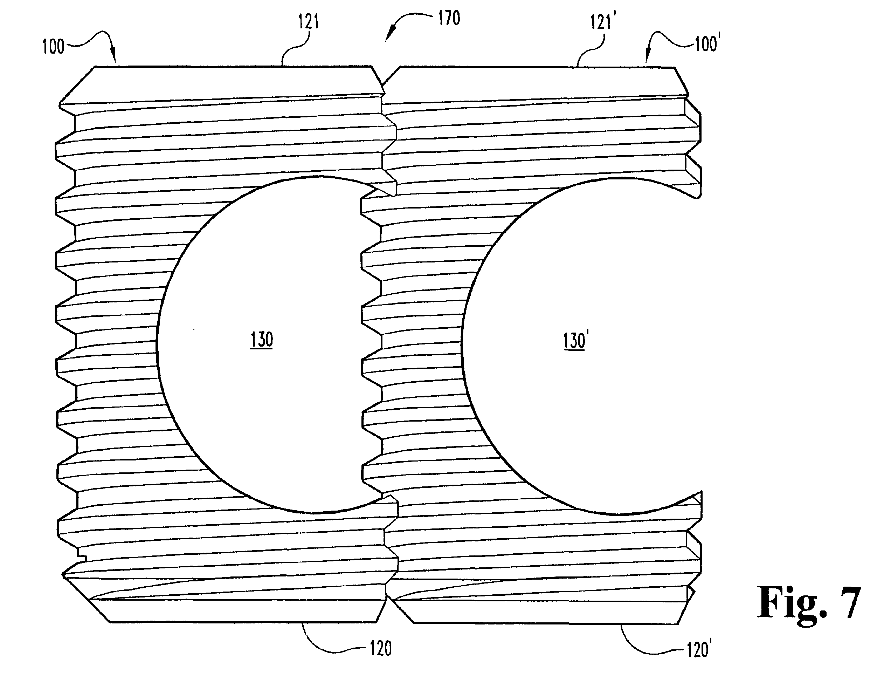

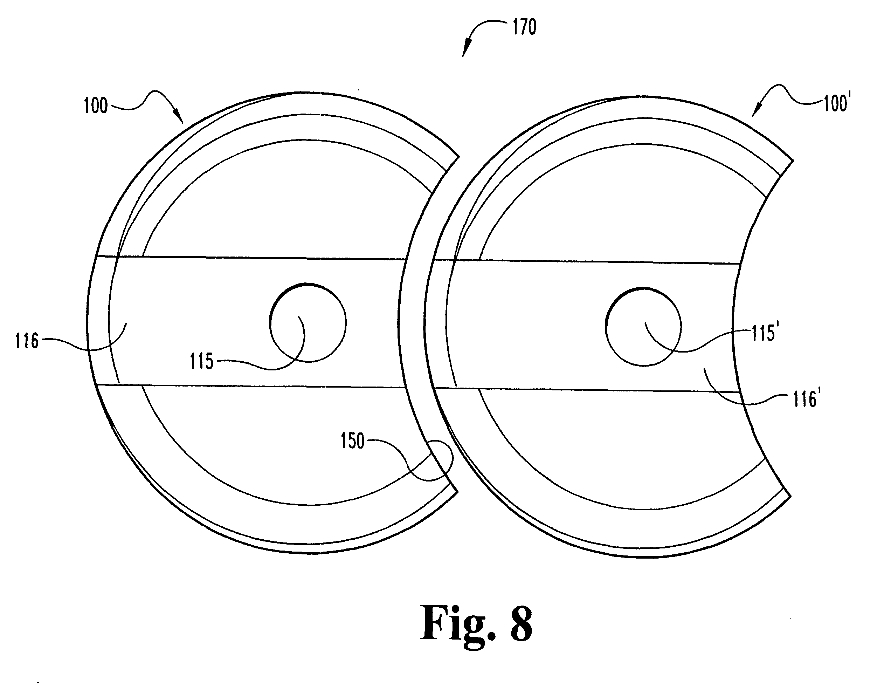

The spacers of implant system 170 may be arranged so that spacers 100 and 100' can nest within each other. As shown in FIGS. 3-5, the spacers may be positioned such that the opening to the cavities, or open chambers, are aligned with each other, thus forming a large, single area for optional placement of osteogenic material. In an alternative embodiment as seen in FIGS. 6-8, spacers 100 and 100' may be positioned so that outer surface 106' of spacer 100' nests within region 170 and 171 (as best seen in Figure 1) formed by ends 150 and 151, respectively.

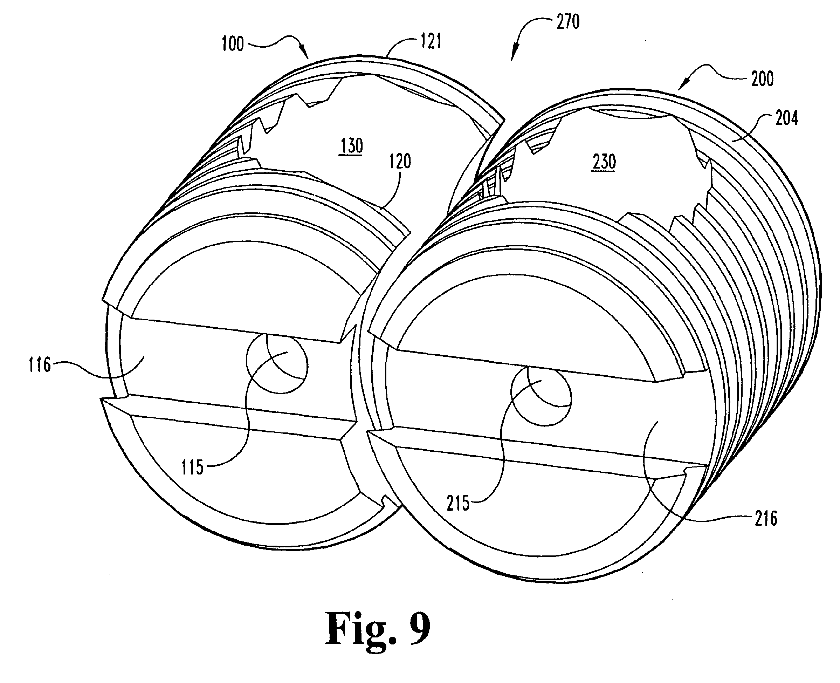

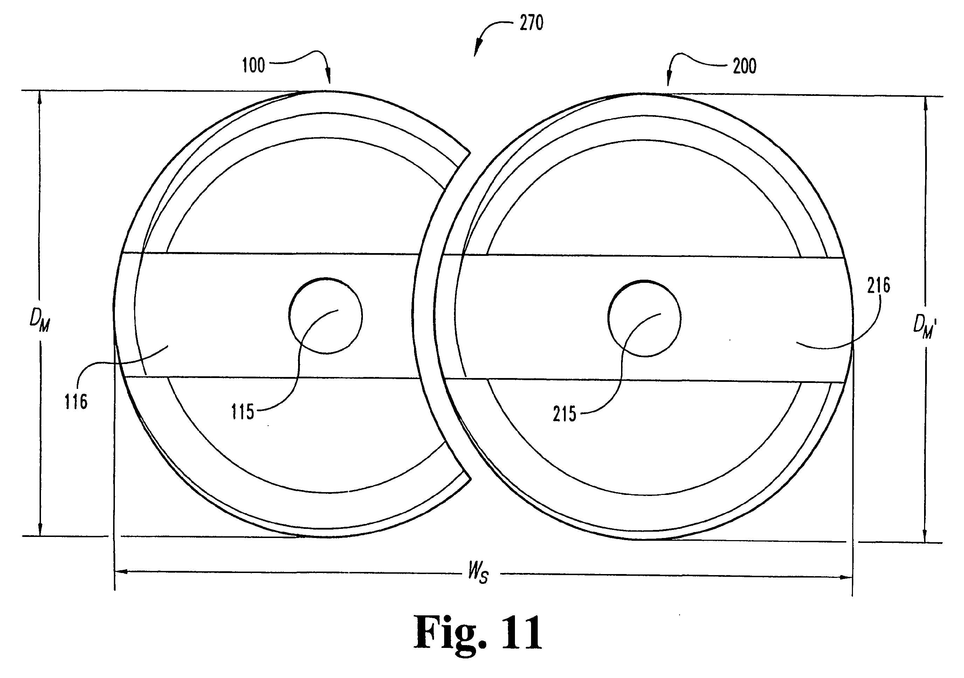

Referring now to FIGS. 9-11, depicted is another implant system 270 of the present invention. System 270 includes a first spacer 100 as described above. Ends 150 and 151 of arms 120 and 121, respectively, of spacer 100 are configured to form a nesting region as described above within which an adjacent spacer can nest to form implant system 270. As seen in the figures, spacer 200, of differing design than spacer 100, having elongated body 204 is nested within spacer 100 to form implant system 270. That is, outer surface 206 of spacer 200 is nested within region 170 and 171 (region 170 and 171 are shown in Figure 1) formed by ends 150 and 151, respectively. In such a nested configuration, there exists a lateral overlap among the structures of the two devices. In this manner, the overall lateral width of the implant system 270 is reduced as compared to one in which two spacers of design of spacer 200 were implanted adjacent to one another. More specifically, it can be seen that implant system 270 has a width Wₛ that is less than the sum of maximum outer diameter D_{M} of spacer 100 and maximum outer diameter D_{M'} of spacer 200. As can also be seen, implant system 270 includes two openings 130 and 230, which can be packed with graft material to promote bone growth through the openings and arthrodesis. A first opening defined by cavity 130 of spacer 100 and external wall of spacer 200, and a second opening is provided by through-hole 230 of spacer 200.

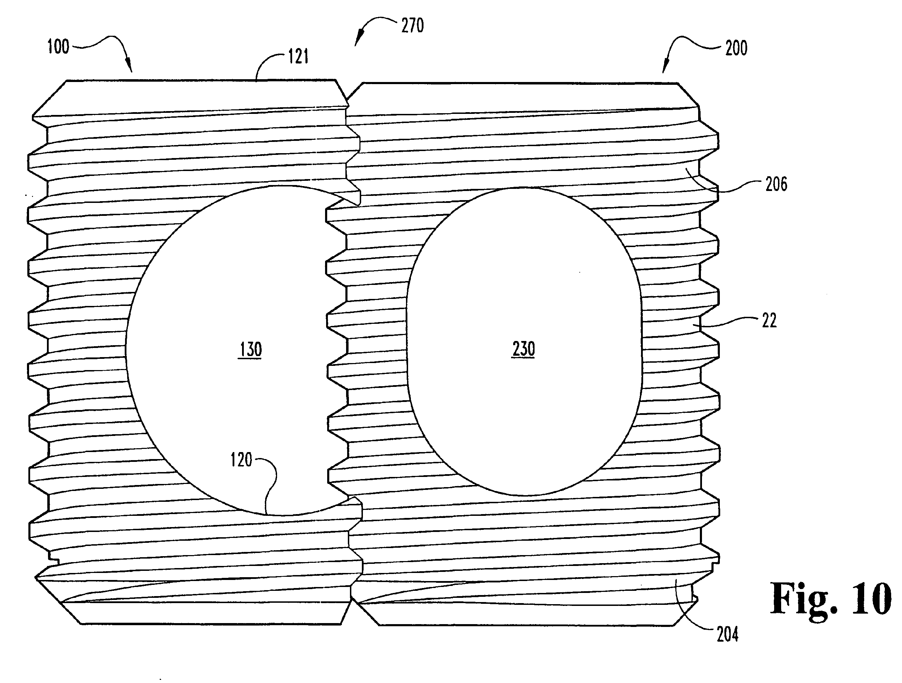

Spacer 200, may be a variety of spacers. For example, spacer 200 may have an outer surface 206, a substantially cylindrical body 22 and a longitudinal axis. The spacer may include a through-hole 230, as discussed above and as depicted in FIGS. 9 and 10. Spacer 200 may also be, for example, a spacer as described in U.S. Patent No. 5,814,084. Thus, spacer 200 can be obtained as a transverse plug from a diaphysis of a long bone having a medullary canal, with the medullary canal forming at least a portion of the through-hole 230 in the spacer 200. As with spacer 100, spacer 200 can include a tool engagement hole 215 and slot 216.

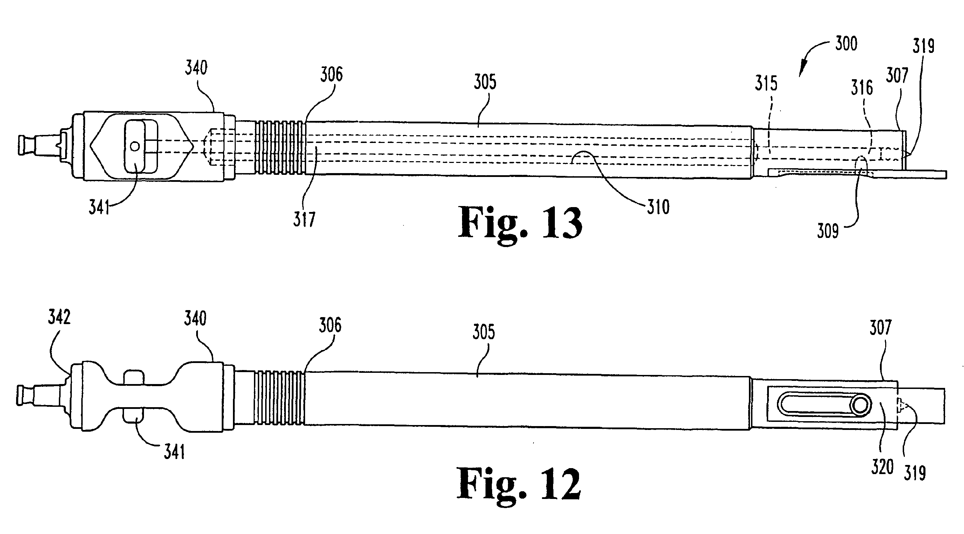

The present invention further contemplates insertion devices for facilitating the implantation of spacers, implants or bone graft. The tools include spacer engaging means for engaging a spacer or other item and occlusion means for blocking an opening defined in the spacer. One embodiment of an insertion tool of this invention is depicted in FIGS. 12-15. Such an insertion tool is a modification of insertion tools as described in International Application Serial No. PCT/US98/11159 filed June 3, 1998, incorporated herein by reference, having additional features as described below.

In one embodiment, an insertion tool 300 is provided which includes a housing 305 having a proximal end 306 and an opposite distal end 307 and defining a passageway 310 between the two ends. A shaft 315 which has a first end 316 and an opposite second end 317 is disposed within the passageway 310. The first end 316 of the shaft 315 is adjacent the distal end 307 of the housing 305. The first end 316 defines a spacer engager 319. An occlusion member 320 is attached to the housing 305.

The spacer engager 319 has any configuration which will engage a spacer. In some embodiments the spacer engager 319 includes a post for engaging a hole in the spacer. The post may have any configuration which will provide for mating engagement with a hole in a spacer. For example, the post may include an engager 319 which is threaded to matingly engage a threaded tool hole. Other embodiments and features may include a sharply pointed tip 319 as shown, or a hexagonal shaped tip. In each case, the engager is shaped and sized to mate engagingly with the tool hole of the spacer. In other embodiments, the spacer engaging means is a pair of prongs having opposite facing spacer engaging members for grasping an outer surface of the spacer.

The spacer insertion tool 300 also includes an occlusion member 320 for blocking an opening defined in the spacer when the spacer engager 319 is engaged to the spacer. In a preferred embodiment, the occlusion member 320 is slideably engaged to the housing 305, and is extendible from the distal end 307 of the housing 305 for blocking an opening in the spacer. The occlusion member 320 closes the mouth and channel defined in the spacer 100.

The tool 300 depicted also includes a handle portion 340. The handle portion includes means for slidingly moving the shaft 315 within the housing 305 and for rotating the shaft 315. In the embodiment shown, the means includes a thumbwheel 341. In some embodiments, the handle portion 340 has a Hudson end attachment 342.

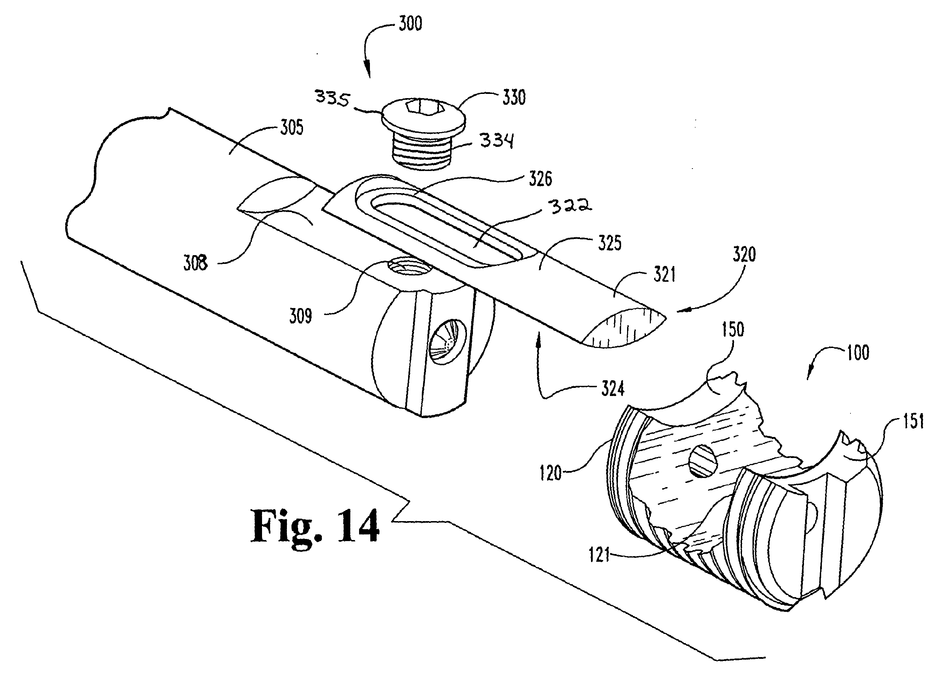

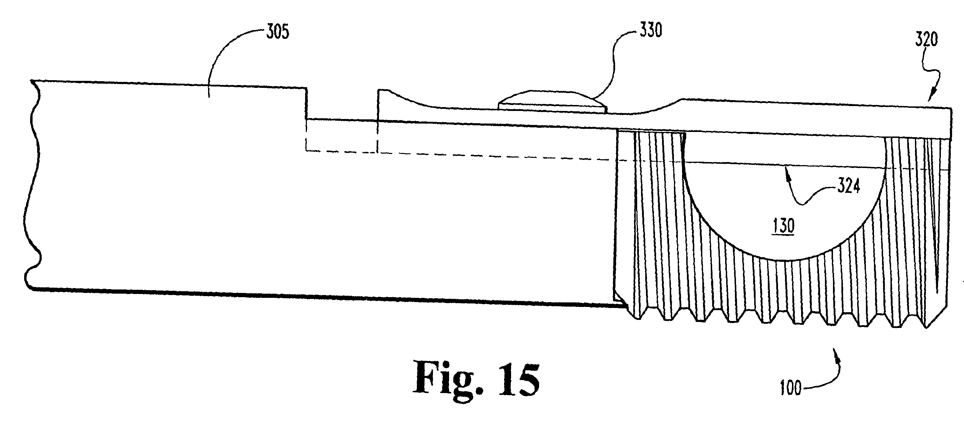

Referring now to Figures 14 and 15, shown are expanded views illustrating an inventive spacer 100 associated with the insertion tool 300 to form an assembly of the invention. As illustrated, insertion tool 300 has an occlusion member 320 having an interior surface 324 which is convexly curved to complement the concave surfaces of ends 150 and 151 of arms 120 and 121, respectively, of spacer 100. Correspondingly, recess 308 of insertion tool 300 has a concave surface complementary to convexly curved surface 324 of occlusion member 320. Further, occlusion member 320 is of a length and design sufficient to span to the distal end of the engaged spacer 100, as depicted in Figure 15. Occlusion member 320 can also have a beveled outer end, or an otherwise smoothed outer end, to facilitate rotary insertion.

Referring now to Figure 14, in one embodiment, the occlusion member 320 includes a plate 321 which defines a groove 322. A fastener 330 is engaged to a fastener bore 309 in the housing 305 and the groove 322 is disposed around the fastener 330. in this way, the plate 321 is slideable relative to the housing 305.

As shown, the housing 305 is preferably provided with a recess 308 which is defined to accept the occlusion member 320 without increasing the effective diameter of the device 300. The occlusion member is also adapted for the best fit with the spacer. For example, the interior surface 324 of the occlusion member would be curved to complement the concave end faces 150 and 151 for engagement. The plate 321 of the occlusion member 320 preferably includes a curved superior surface 325 which approximates and completes the minor diameter of the dowel 100 when the spacer engager 319 is engaged to the tool engaging hole and the occlusion member 320 is blocking the channel of the spacer 100. Preferably, the plate 321 and the arms 120 and 121 of the spacer 100 will be configured such that when the tool 300 is engaged to the spacer 100, the curved superior surface 325 will not increase the effective root diameter RD of the threaded outer surface of the spacer 100. This facilitates rotation and screw insertion of the spacer and occlusion member combination into an intervertebral space.

In preferred embodiments, as shown in Figures 14 and 15, the plate 321 defines a recess 326 surrounding a groove 322. A fastener 330 is engaged to a fastener bore 309 in the housing 305 and the groove 322 is disposed around the fastener 330. In this way, the plate 321 is slideable relative to the housing 305. The fastener 330 is preferably provided with a post 334, and a plate engaging means or head portion 335. The fastener 330 preferably includes an internal hex as shown for receiving a fastener driving tool. The post portion 334 may be threaded for mating engagement with threaded bore 309 in the housing 305. In preferred embodiments, the diameter of the head portion 335 is greater than the diameter of the post 334. The diameter of the post 334 is less than the width of the groove 322. The diameter of the head portion is greater than width of the groove 322 but preferably no greater than the distance between the outer edges of the recess 326. Thus, the head portion 335 of the fastener 330 can rest on the recess 326 while the post portion 334 extends through the groove 322. In this way, plate 321 is slidable relative to the housing 305. This also provides for a low profile device which can be inserted into various cannula for percutaneous procedures.

Although any open-chambered spacers having the inventive features described herein is contemplated, in one embodiment the spacers are obtained as an off-center transverse plug from the diaphysis of a long bone. This results in a dowel having an open-mouthed chamber. Because the long bone naturally includes the medullary canal, a pre-formed chamber is inherently contained within the dowel. When the plug is cut off-center in a certain way, the dowel includes an open-mouthed chamber.

Other surface features can be defined along the length of the spacer. The surface features can provide engaging surfaces to facilitate engagement with the vertebrae and prevent slippage of the spacer as is sometimes seen with a smooth graft. The surface feature may include, for example, a groove or stop rib inscribed along the circumference of the spacer. In some embodiments, also, the upper and lower walls of the spacers of the invention may include flattened portions to stabilize the dowel by neutralizing any rotational torque that may be induced by pressure on the sidewall.

For cervical fusions, dowel spacers of the invention are preferably obtained from the fibula, radius, ulna or humerus. The dimensions of such dowels are typically between about 8-15mm in length or depth and about 10-14mm in diameter. For thoracic and lumbar fusions, the dowel is preferably obtained from the humerus, femur or tibia. The dimensions of such dowels are typically between about 10-30mm in length and about 14-20mm in diameter.

The chamber may be packed with any suitable osteogenic material. In a preferred embodiment, the osteogenic composition has a length which is greater than the length of the chamber so that the osteogenic composition will contact the endplates of the adjacent vertebrae when the spacer is implanted within the vertebrae. This provides better contact of the composition with the endplates to stimulate bone ingrowth.

Any suitable osteogenic material or composition is contemplated, including autograft, allograft, xenograft, demineralized bone, synthetic and natural bone graft substitutes, such as bioceramics and polymers, and osteoinductive factors. The terms osteogenic material or osteogenic composition used here means virtually any material that promotes bone growth or healing including autograft, allograft, xenograft, bone graft substitutes and natural, synthetic and recombinant proteins, hormones and the like.

Autograft can be harvested from locations such as the iliac crest using drills, gouges, curettes and trephines and other tools and methods which are well known to surgeons in this field. Preferably, autograft is harvested from the iliac crest with a minimally invasive donor surgery. The osteogenic material may also include bone reamed away by the surgeon while preparing the end plates for the spacer.

Advantageously, where autograft is chosen as the osteogenic material, only a very small amount of bone material is needed to pack the chamber. The autograft itself is not required to provide structural support as this is provided by the spacer. The donor surgery for such a small amount of bone is less invasive and better tolerated by the patient. There is usually little need for muscle dissection in obtaining such small amounts of bone. The present invention therefore eliminates or minimizes many of the disadvantages of employing autograft.

Natural and synthetic graft substitutes which replace the structure or function of bone are also contemplated for the osteogenic composition. Any such graft substitute is contemplated, including for example, demineralized bone matrix, mineral compositions and bioceramics. As is evident from a review of An Introduction to Bioceramics, edited by Larry L. Hench and June Wilson (World Scientific Publishing Co. Ptd. Ltd, 1993, volume 1), there is a vast array of bioceramic materials, including BIOGLASS*, hydroxyapatite and calcium phosphate compositions known in the art which can be used to advantage for this purpose. That disclosure is herein incorporated by reference for this purpose. Preferred calcium compositions include bioactive glasses, tricalcium phosphates and hydroxyapatites. In one embodiment, the graft substitute is a biphasic calcium phosphate ceramic including tricalcium phosphate and hydroxyapatite.

In some embodiments, the osteogenic compositions used in this invention comprise a therapeutically effective amount to stimulate or induce bone growth of a substantially pure bone inductive or growth factor or protein in a pharmaceutically acceptable carrier. The preferred osteoinductive factors are the recombinant human bone morphogenetic proteins (rhBMPs) because they are available in unlimited supply and do not transmit infectious diseases. Most preferably, the bone morphogenetic protein is a rhBMP-2, rhBMP-4 or heterodimers thereof.

Recombinant BMP-2 can be used at a concentration of about 0.4 mg/ml to about 1.5 mg/ml, preferably near 1.5 mg/ml. However, any bone morphogenetic protein is contemplated including bone morphogenetic proteins designated as BMP-1 through BMP-13. BMPs are available from Genetics Institute, Inc., Cambridge, Massachusetts and may also be prepared by one skilled in the art as described in U.S. Patent Nos. 5,187,076 to Wozney et al.; 5,366,875 to Wozney et al.; 4,877,864 to Wang et al.; 5,108,922 to Wang et al.; 5,116,738 to Wang et al.; 5,013,649 to Wang et al.; 5,106,748 to Wozney et al.; and PCT Patent Nos. WO93/00432 to Wozney et al.; WO94/26893 to Celeste et al.; and WO94/26892 to Celeste et al. All osteoinductive factors are contemplated whether obtained as above or isolated from bone. Methods for isolating bone morphogenetic protein from bone are described in U.S. Patent No. 4,294,753 to Urist and Urist et al., 81 PNAS 371, 1984.

The choice of carrier material for the osteogenic composition is based on biocompatibility, biodegradability, mechanical properties and interface properties as well as the structure of the load bearing member. The particular application of the compositions of the invention will define the appropriate formulation. Potential carriers include calcium sulphates, polylactic acids, polyanhydrides, collagen, calcium phosphates, polymeric acryiic esters and demineralized bone. The carrier may be any suitable carrier capable of delivering the proteins. Most preferably, the carrier is capable of being eventually resorbed into the body. One preferred carrier is an absorbable collagen sponge marketed by Integra LifeSciences Corporation under the trade name Helistat* Absorbable Collagen Hemostatic Agent. Another preferred carrier is a biphasic calcium phosphate ceramic. Ceramic blocks are commercially available from Sofamor Danek Group, B. P. 4-62180 Rang-du-Fliers, France and Bioland, 132 Rou d Espangne, 31100 Toulouse, France. The osteoinductive factor is introduced into the carrier in any suitable manner. For example, the carrier may be soaked in a solution containing the factor.

The present invention also provides methods for making the open spacers of this invention. In one embodiment, a method for making an open chambered bone dowel includes obtaining an off-center plug from the diaphysis of a long bone so that the dowel has an open chamber, and machined to have inventive features as described herein, for example to have concave surfaces on each end configured to nest with an adjacent spacer, as described above. The open chamber is preferably substantially concave or C-shaped and has an axis that is substantially perpendicular to the long axis of the dowel. Appropriate human source bones include the femur, tibia, fibula, humerus, radius and ulna. Long bones from other species are also contemplated although human source bones are generally preferred for human recipients.

The first step is to identify an acceptable donor based upon appropriate standards for the particular donor and recipient. For example, where the donor is human, some form of consent such as a donor card or written consent from the next of kin is required. Where the recipient is human, the donor must be screened for a wide variety of communicable diseases and pathogens, including human immunodeficiency virus, cytomegalovirus, hepatitis B, hepatitis C and several other pathogens. These tests may be conducted by any of a number of means conventional in the art, including but not limited to ELISA assays, PCR assays, or hemagglutination. Such testing follows the requirements of: (i) American Association of Tissue Banks, Technical Manual for Tissue Banking, Technical Manual- Musculoskeletal Tissues, pages M19-M20; (ii) The Food and Drug Administration, Interim Rule, Federal Register/Vol. 58, No. 238/Tuesday, December 14, 1994/Rules and Regulations/65517, D. Infectious Disease Testing and Donor Screening; (iii) MMWR/Vol. 43/No. RR-8m Guidelines for Preventing Transmission of Human Immunodeficiency Virus Through Transplantation of Human Tissue and organs, pages 4-7; (iv) Florida Administrative Weekly, Vol. 10, No. 34, August 21, 1992, 59A-1.001-01459A-1.005(12)(c), F.A.C., (12)(a)-(h), 59A-1.005 (15), F.A.C., (4)(a)-(8). In addition to a battery of standard biomechanical assays, the donor, or their next of kin, is interviewed to ascertain whether the donor engaged in any of a number of high risk behaviors such as having multiple sexual partners, suffering from hemophilia, engaging in intravenous drug use, etc. Once a donor has been ascertained to be acceptable, the bones useful for obtention of the dowels are recovered and cleaned.

Preferably, the bone plugs are obtained using a diamond or hard metal tipped cutting bit which is water cleaned and cooled. Commercially available bits (e.g. core drills) having a generally circular nature and an internal vacant diameter between about 10mm to about 20 mm are amenable to use for obtention of these bone plugs. Such core drills are available, for example, from Starlite, Inc. In one embodiment, a pneumatic driven miniature lathe having a spring loaded carriage which travels parallel to the cutter is used. The lathe has a drive system which is a pneumatic motor with a valve controller which allows a desired RPM to be set. The carriage rides on two runners which are 1.0 inch stainless rods and has travel distance of approximately 8.0 inches. One runner has set pin holes on the running rod which will stop the carriage from moving when the set pin is placed into the desired hole. The carriage is moveable from side to side with a knob which has graduations for positioning the graft. A vice on the carriage clamps the graft and holds it in place while the dowel is being cut. The vice has a cut-out area in the jaws to allow clearance for the cutter.

In operation, the carriage is manually pulled back and locked in place with a set pin. The graft is loaded into the vice and is aligned with the cutter. Sterile water is used to cool and remove debris from graft and/or dowel as the dowel is being cut. The water travels down through the center of the cutter to irrigate as well as clean the dowel under pressure. After the dowel is cut, sterile water is used to eject the dowel out of the cutter.

Dowels of any size can be prepared according to this invention. In some embodiments, the dowels range from 5mm to 30mm diameters with lengths of about 8mm to about 36mm being generally acceptable, although other appropriate gradations in length and diameter are available. For cervical dowels, such as anterior cervical fusion or ACF dowels, lengths of 8mm, 9mm, up to about 15mm are generally desirable. Dowels of differing diameter are most conveniently obtained as follows:

| Diameter | Source |
|---|---|
| 10.6-11 mm | fibula |
| 12mm | radius |
| 14mm | ulna |
| 14+mm | small humeri |

Dowels for thoracic and lumbar fusions, such as anterior thoracic inner body fusion (ATIF) and anterior lumbar inner body fusion (ALIF) dowels, respectively, having a depth of between about 10-36 mm, and preferably between about 15-24mm, are generally acceptable, depending on the needs of a particular patient. Dowels of differing diameter for thoracic and lumbar fusions are most conveniently obtained as follows:

| Diameter | Source |
|---|---|
| 14-16mm | humerus |
| 16-18mm | femur |
| 18-20mm | tibia |

While the foregoing diameters and source bones for such dowels is a useful guide, one of the significant advances provided by this invention is that the open-chambered dowel of this invention provides tremendous flexibility with respect to the source bone used.

Since the spacers of the preferred embodiment are obtained from off-center transverse plugs across the diaphysis of long bones, each dowel has the feature of having a substantially "C"-shaped chamber through the dowel perpendicular to the length of the dowel formed by the intersection of the natural intramedullary canal of the source bone and the cutter blade as it forms the plug. The canal cavity in the long bone is, in vivo, filled with bone marrow. In the standard Cloward Dowel and unicortical dowels known in the art, no such natural cavity exists and the cancellous bone that forms the body of such dowels tends to be too brittle to accept machining of such a cavity. The dowels of this invention, by the nature of their origin, inherently define such a cavity. Naturally, based on this disclosure, those skilled in the art will recognize that other bone sources could be used which do not have the intramedullary canal, and if sufficient strength is inherent to the bone, a cavity or chamber could be machined. In addition, it will be appreciated from the instant disclosure that an existing diaphysial cortical dowel having a through hole, such as that depicted in U.S. Patent No. 5,814,084, could be modified by machining one side of such a dowel until one wall of the dowel is sufficiently abraded to "break-through", thereby transforming the diaphysial cortical dowel into a "C"-shaped dowel, and machined to incorporate the features of this invention. Accordingly, such extensions of this invention should be considered as variants of the invention disclosed herein and therefore come within the scope of the claims appended hereto.

The marrow is preferably removed from the intramedullary canal of the diaphysial plugs and the cavity is cleaned, leaving the chamber. The spacer may be provided to the surgeon with the chamber prepacked or empty for the surgeon to pack during surgery. The cavity or chamber can then be packed with an osteogenic material or composition.

The plug is then machined, preferably in a class 10 clean room to the dimensions and to have the features desired. The machining is preferably conducted on a lathe such as a jeweler's lathe, or machining tools may be specifically designed and adapted for this purpose. Specific tolerances for the dowels and reproducibility of the product dimensions are important features for the successful use of such dowels in the clinical setting.

In some embodiments, the forward end of the dowel which is to be inserted into a cavity formed between adjacent vertebrae is chamfered. The curvature of the chamfered end facilitates insertion of the dowel into the intervertebral space. Chamfering can be accomplished by appropriate means such as by machining, filing, sanding or other abrasive means. The tolerance for the chamfering is fairly liberal and the desired object is merely to round or slightly point the end of the dowel that is to be inserted into the cavity formed between adjacent vertebrae to be fused.

In some embodiments, the invention includes methods for providing surface features into the walls of the dowels. The methods may include defining a tool or instrument attachment hole in an end of the dowel. The hole may be drilled and preferably tapped. Preferably, the dowel will be of such dimensions as to fit standard insertion tools, such as those produced by Sofamor Danek Group, Inc. (1800 Pyramid Place, Memphis, TN 38132, (800) 933-2635). In addition, a score mark or driver slot may be inscribed on the instrument attachment end of the dowel so that the surgeon can align the dowel so that the chamber is parallel with the length of the recipient's spinal column. The mark or slot allows the surgeon to orient the dowel properly after the dowel is inserted and the chamber is no longer visible. In the proper orientation, the endplates of the adjacent vertebrae are exposed to osteogenic material in the chamber. In some embodiments, the driver slot is omitted to preserve as much bone stock, and therefore strength, in the end as possible.

Surface features such as grooves and threads may be preferably defined or inscribed on the outer cylindrical surface of the dowel. Machining of such features on dowels known in the art is difficult if not impossible due to the brittle cancellous nature of such dowels. Accordingly, the dowels of this invention have the advantage of having very good biomechanical properties amenable to such machining.

Those skilled in the art will also recognize that any of a number of different means may be employed to produce the threaded or grooved embodiments of the dowel of this invention. However, one preferred embodiment of a thread cutter is described in International Application Serial No. PCT/US98/11159 filed June 3, 1998 entitled OPEN INTERVERTEBRAL SPACERS, herein incorporated by reference. The final machined product may be stored, frozen or freeze-dried and vacuum sealed as known in the art for later use.

The spacers and tools in this invention can be conveniently incorporated into known surgical, preferably minimally invasive, procedures. The spacers of this invention can be inserted using laparoscopic technology as described in Sofamor Danek USA's Laparoscopic Bone Dowel Surgical Technique, 1995, 1800 Pyramid Place, Memphis, Tennessee 38132, 1-800-933-2635, preferably in combination with the insertion tool 300 of this invention. Spacers of this invention can be conveniently incorporated into Sofamor Danek's laparoscopic bone dowel system that facilitates anterior interbody fusions with an approach that is much less surgically morbid than the standard open anterior retroperitoneal approaches. This system includes templates, trephines, dilators, reamers, ports and other devices required for laparoscopic dowel insertion. Alternatively, a minimally invasive open anterior approach using Sofamor Danek's open anterior bone dowel instrumentation or a posterior surgical approach using Sofamor Danek's posterior approach bone dowel instrumentation are contemplated.

The present invention also includes methods for fusing adjacent vertebrae. The spine may be approached from any direction indicated by the circumstances. The vertebrae and the intervertebral space are prepared according to conventional procedures to receive the spacer. A spacer of the appropriate dimensions is selected by the surgeon, based on the size of the cavity created and the needs of the particular patient undergoing the fusion. The spacer is mounted on an instrument, preferably via an instrument attachment hole. In one embodiment, an osteogenic material is placed within the chamber of the spacer and the channel and or mouth of the spacer is then blocked with an occlusion member of the instrument. The spacer is then inserted into the cavity created between the adjacent vertebra to be fused. The spacer is oriented within the intervertebral space so the osteogenic material in the chamber is in communication with the end plates of the vertebra. Once the spacer is properly oriented within the intervertebral space, the occlusion member of the instrument can be withdrawn from the spacer aperture and the spacer engager is disengaged from the spacer.

In some embodiments, osteogenic material is packed into the chamber through the channel after implantation. In still other embodiments, a second spacer is implanted into the intervertebral space.

While the foregoing description discloses specific aspects of this invention, those skilled in the art will recognize that any of a number of variations on the basic theme disclosed herein can be made. It is contemplated that the spacers of this invention can be formed of any suitable biocompatible material, including metals, ceramics, polymers, composites, alloys and the like. Some embodiments include titanium, stainless steel, and Hedrocel^{®}. Thus, for example, differing shapes can be made from the diaphysis of various bones and can be used for orthopaedic purposes other than vertebral fusions. In addition, any of a number of known bone treatments can be applied to the dowel of this invention to alter its properties. For example, the methods disclosed in U.S. Patent Nos. 4,627,853; 5,053,049; 5,306,303 and 5,171,279 can be adapted and applied to the invention disclosed herein. Accordingly the disclosures of those patents are herein incorporated by reference for this purpose.

It should be understood that the example and embodiments described herein are for illustrative purposes only and that various modifications or changes in light thereof will be suggested to persons skilled in the art and are to be included within the scope of the invention as defined in the appended claims.

## Claims

1. An interbody fusion spacer (100), comprising:
an elongated body (105) defining a chamber (130), said body having a first arm (120) and a second opposing arm (121), said arms defining an opening (110) in communication with said chamber (130), **characterised in that** each of said first arm (120) and second arm (121) has an end (150, 151) configured to form a nesting region (170, 171) within which an adjacent spacer can nest.

2. The spacer of claim 1, wherein said body (105) further comprises a tool engaging end (101) defining a tool engaging hole (115) for receiving a driving tool for implanting the spacer.

3. The spacer of claim 1, wherein said elongated body (105) is comprised of a porous material.

4. The spacer of claim 1, wherein said porous material is bone.

5. The spacer of claim 4, wherein said bone is cortical bone.

6. The spacer of claim 4, wherein said bone is obtained as a transverse cut from the diaphysis of a long bone having a medullary canal.

7. The spacer of claim 1, wherein said body (105) has an outer surface that defines threaded bone-engaging portions (107).

8. The spacer of claim 1, wherein each of said ends (150, 151) has a concave surface.

9. The spacer of claim 8, wherein the concave surface extends to an outer edge of the spacer (100).

10. The spacer of claim 1, further comprising an osteogenic material disposed within said channel.

11. The spacer of claim 10, wherein said osteogenic material comprises natural bone, demineralized bone, a calcium phosphate material, a bioceramic, bioglass, an osteoinductive factor and mixtures thereof.

12. The spacer of claim 10, wherein said osteogenic material comprises a bone morphogenetic protein.

13. The spacer of claim 12, wherein said bone morphogenetic protein comprises a recombinant protein.

14. The spacer of claim 13, wherein said recombinant bone morphogenetic protein comprises a human protein.

15. The spacer of claim 14, wherein said recombinant human protein comprises BMP-2, BMP-4 or heterodimers thereof.

16. The spacer of claim 1, wherein said ends of each of said arms (120, 121) has an arcuate surface configured to receive a convex surface of an adjacent spacer.

17. The spacer of claim 1, wherein the nesting region (170, 171) is arcuately truncated.

18. The spacer of claim 1, in combination with an adjacent, second spacer, wherein said elongated body (105) has a first maximum outer diameter and each of said arms (120, 121) of said elongated body are configured to form a nesting region for nesting of said adjacent second spacer that has a second maximum outer diameter to form a spacer assembly having a width less than the sum of the combined maximum outer diameters.

19. The spacer of claim 8, which is formed of bone.

20. The spacer of claim 1, wherein said elongated body (105) has a longitudinal axis and said chamber (130) extends perpendicular to said longitudinal axis.

21. An interbody fusion spacer according to claim 1, wherein:
said elongated body (105) is of bone having a longitudinal axis and defining said chamber (130) extending perpendicular to said longitudinal axis of said body, said body of bone comprised of bone from the diaphysis of a long bone having a medullary canal, and said nested region (170, 171) having a concave surface.

22. The spacer of claim 21, wherein said body further comprises a tool engaging end (101) defining a tool engaging hole (115) for receiving a driving tool for implanting the spacer.

23. An interbody fusion spacer according to claim 1 in combination with:
a second interbody fusion spacer (100', 200) having a second elongated (105', 204) body of bone and an outer surface (206), said second interbody fusion spacer (100', 200) nestable within said first interbody fusion spacer (100) within said nested regions (170, 171).

24. The spacer of claim 23, wherein said second elongated body (204) defines a through-hole (230).

25. The spacer of claim 24, wherein said second elongated body (204) has a longitudinal axis and said through-hole (230) extends perpendicular to said longitudinal axis.

26. The spacer of claim 23, wherein said second elongated body defines a second chamber, said second body having a third arm and a fourth opposing arm, said third and fourth arms defining a second opening in communication with said second chamber, said second interbody fusion spacer nestable within said first interbody fusion spacer.

27. The spacer of claim 26, said system further comprising an osteogenic material disposed in at least one of said chambers of said spacers.

28. An interbody fusion spacer combination, comprising:
a first interbody fusion spacer (100) according to any one of claims 1 to 22 and
a second spacer (100', 200) adjacent to said first spacer (100);
said second spacer (100', 200) having a second maximum outer diameter, said second spacer in nested relationship with said first spacer by having a first portion nested within a region on said first arm (120) of said first spacer and a second portion nested within a region of said second arm (121) of said first spacer;
said interbody fusion spacer combination, by virtue of said nested relationship, having an overall width that is less than the sum of said first maximum outer diameter and said second maximum outer diameter.

## Patentansprüche

1. Zwischenkörperfusionsdistanzstück (100), das aufweist:
einen länglichen Körper (105), der eine Kammer (130) definiert, wobei der Körper einen ersten Arm (120) und einen zweiten entgegengesetzten Arm (121) aufweist, wobei die Arme eine Öffnung (110) in Verbindung mit der Kammer (130) definieren, **dadurch gekennzeichnet, dass** ein jeder von erstem Arm (120) und zweitem Arm (121) ein Ende (150, 151) aufweist, das so ausgebildet ist, dass ein Ineinandersteckbereich (170, 171) gebildet wird, innerhalb dessen ein benachbartes Distanzstück ineinandergesteckt werden kann

2. Distanzstück nach Anspruch 1, bei dem der Körper (105) außerdem ein Werkzeugeingriffsende (101) aufweist, das ein Werkzeugeingriffsloch (115) für das Aufnehmen eines Treibwerkzeuges für das Implantieren des Distanzstückes definiert.

3. Distanzstück nach Anspruch 1, bei dem der längliche Körper (105) ein poröses Material aufweist.

4. Distanzstück nach Anspruch 1, bei dem das poröse Material Knochen ist.

5. Distanzstück nach Anspruch 4, bei dem der Knochen ein kortikaler Knochen ist.

6. Distanzstück nach Anspruch 4, bei dem der Knochen als ein Querschnitt aus der Diaphysis eines langen Knochens mit einem Markkanal erhalten wird.

7. Distanzstück nach Anspruch 1, bei dem der Körper (105) eine Außenfläche aufweist, die Gewindeknocheneingriffsabschnitte (107) definiert.

8. Distanzstück nach Anspruch 1, bei dem ein jedes der Enden (150, 151) eine konkave Fläche aufweist.

9. Distanzstück nach Anspruch 8, bei dem sich die konkave Fläche bis zu einem äußeren Rand des Distanzstückes (100) erstreckt.

10. Distanzstück nach Anspruch 1, das außerdem ein osteogenes Material aufweist, das innerhalb des Kanals angeordnet ist.

11. Distanzstück nach Anspruch 10, bei dem das osteogene Material natürlichen Knochen, entmineralisierten Knochen, ein Calciumphosphatmaterial, ein Biokeramikmaterial, Bioglas, einen osteoinduktiven Faktor und deren Mischungen aufweist.

12. Distanzstück nach Anspruch 10 bei dem das osteogene Material ein knochenmorphogenetisches Protein aufweist.

13. Distanzstück nach Anspruch 12, bei dem das knochenmorphogenetische Protein ein rekombiniertes Protein aufweist,

14. Distanzstück nach Anspruch 13, bei dem das rekombinierte knochcumorphogenetische Protein ein menschliches Protein aufweist,

15. Distanzstück nach Anspruch 14, bei dem das rekombinierte menschliche Protein BMP-2, BMP-4 oder deren Heterodimere aufweist.

16. Distanzstück nach Anspruch 1, bei dem die Enden eines jeden der Arme (120, 121) eine bogenförmige Fläche aufweisen, die so ausgebildet ist, dass sie eine konvexe Fläche eines benachbarten Distanzsstückes aufnimmt.

17. Distanzstück nach Anspruch 1, bei dem der Ineinandersteckbereich (170, 171) bogenförmig abgestumpft ist.

18. Distanzstück nach Anspruch 1 in Kombination mit einem benachbarten zweiten Distanzstück, bei dem der längliche Körper (105) einen ersten maximalen Außendurchmesser aufweist und jeder der Arme (120. 121) des länglichen Körpers ausgebildet ist, um einen Ineinandersteckbereich für das Ineinanderstecken des benachbarten zweiten Distanzstückes zu bilden, das einen zweiten maximalen Außendurchmesser aufweist, um eine Distanzstückanordnung mit einer Breite zu bilden, die kleiner ist als die Summe der kombinierten maximalen Außendurchmesser.

19. Distanzstück nach Anspruch 8, das aus Knochen gebildet wird.

20. Distanzstück nach Anspruch 1, bei dem der längliche Körper (105) eine Längsachse aufweist und sich die Kammer (130) senkrecht zur Längsachse erstreckt.

21. Zwischenkörperfusionsdistanzstück nach Anspruch 1, bei dem:
der längliche Körper (105) aus Knochen besteht, wobei er eine Längsachse aufweist und die Kammer (130) definiert, die sich senkrecht zur Längsachse des Körpers erstreckt, wobei der Knochenkörper Knochen aus der Diaphysis eines langen Knochens mit einem Markkanal aufweist, und wobei der Incinandersteckbereicht (170, 171) eine konkave Fläche aufweist.

22. Distanzstück nach Anspruch 21, bei dem der Körper außerdem ein Werkzeugeingriffsende (101) aufweist, das ein Werkzeugeingriffsloch (115) für das Aufnehmen eines Treibwerkzeuges für das Implantieren des Distanzstückes definiert.

23. Zwischenkörperfusionsdistanzstück nach Anspruch 1 in Kombination mit:
einem zweiten Zwischenkörperfusionsdistanzstück (100', 200) mit einem zweiten länglichen Knochenkörper (105', 204) und einer Außenfläche (206), wobei das zweite Zwischenkörperfusionsdistanzstück (100', 200) innerhalb des ersten Zwischenkörperfusionsdistanzstückes (100) innerhalb der Ineinandersteckbereiche (170, 171) ineinandergesteckt werden kann.

24. Distanzstück nach Anspruch 23, bei dem der zweite längliche Körper (204) ein Durchgangsloch (230) definiert.

25. Distanzstück nach Anspruch 24, bei dem der zweite längliche Körper (204) eine Längsachse aufweist und sich das Durchgangsloch (230) senkrecht zur Längsachse erstreckt.

26. Distanzstück nach Anspruch 23, bei dem der zweite längliche Körper eine zweite Kammer definiert, wobei der zweite Körper einen dritten und einen vierten entgegengesetzten Arm aufweist, wobei der dritte und der vierte Arm eine zweite Öffnung in Verbindung mit der zweiten Kammer definieren, wobei das zweite Zwischenkörperfusionsdistanzstück innerhalb des ersten Zwischenkörperfusionsdistanzstückes ineinandergesteckt werden kann.

27. Distanzstück nach Anspruch 26, wobei das System außerdem ein osteogenes Material aufweist, das in mindestens einer der Kammern der Distanzstücke angeordnet ist.

28. Zwischenkörperfusionsdistanzstückkombination, die aufweist:
ein erstes Zwischenkörperfusionsdistanzstück (100) nach einem der Ansprüche 1 bis 22; und
ein zweites Distanzstück (100', 200) benachbart dem ersten Distanzstück (100);
wobei das zweite Distanzstück (100', 200) einen zweiten maximalen Außendurchmesser aufweist, wobei das zweite Distanzstück in einer ineinandergesteckten Beziehung zum ersten Distanzstück ist, indem es einen ersten Abschnitt, der innerhalb eines Bereiches am ersten Arm (120) des ersten Distanzstückes ineinandergesteckt ist, und einen zweiten Abschnitt aufweist, der innerhalb eines Bereiches des zweiten Armes (121) des ersten Distanzstückes ineinandergesteckt ist;
wobei die Zwischenkörperfusionsdistanzstückkombination infolge der ineinandergesteckten Beziehung eine Gesamtbreite aufweist, die kleiner ist als die Summe des ersten maximalen Außendurchmessers und des zweiten maximalen Außendurchmessers.

## Revendications

1. Ecarteur de fusion intervertébrale (100), comprenant:
un corps allongé (105) définissant une chambre (130), ledit corps comportant un premier bras (120) et un deuxième bras opposé (121), lesdits bras définissant une ouverture (110), en communication avec ladite chambre (130), **caractérisé en ce que** chacun desdits premier (120) et deuxième (121) bras comporte une extrémité (150, 151) configurée de sorte à former une région à emboîtement (170, 171) dans laquelle peut être emboîté un écarteur adjacent.

2. Ecarteur selon la revendication 1, dans lequel ledit corps (105) comrpend en outre une extrémité d'engagement d'un outil (101) définissant un trou d'engagement d'un outil (115) pour recevoir un outil d'enfoncement afin d'implanter l'écarteur.

3. Ecarteur selon la revendication 1, dans lequel ledit corps allongé (105) est composé d'un matériau poreux.

4. Ecarteur selon la revendication 1, dans lequel ledit matériau poreux est de l'os.

5. Ecarteur selon la revendication 4, dans lequel ledit os est un os cortical.

6. Ecarteur selon la revendication 4, dans lequel ledit os est prélevé sous forme d'une découpe transversale de la diaphyse d'un os long comportant un canal médullaire.

7. Ecarteur selon la revendication 1, dans lequel ledit corps (105) comporte une surface externe définissant des parties filetées d'engagement de l'os (107).

8. Ecarteur selon la revendication 1, dans lequel chacune desdites extrémités (150, 151) comporte une surface concave.

9. Ecarteur selon la revendication 8, dans lequel la surface concave s'étend vers un bord externe de l'écarteur (100)

10. Ecarteur selon la revendication 1, comprenant en outre un matériau ostéogène disposé dans ledit canal.

11. Ecarteur selon la revendication 10, dans lequel ledit matériau ostéogène comprend un os naturel, un os déminéralisé, un matériau de phosphate de calcium, une biocéramique, un facteur ostéoinducteur et des mélanges de ceux-ci.

12. Ecarteur selon la revendication 10, dans lequel ledit matériau ostéogène comprend une protéine morphogénétique osseuse.

13. Ecarteur selon la revendication 12, dans lequel ladite protéine morphogénétique osseuse comprend une protéine recombinante.

14. Ecarteur selon la revendication 13, dans lequel ladite protéine morphogénétique osseuse comprend une protéine humaine,

15. Ecarteur selon la revendication 14, dans lequel ladite protéine recombinante comprend une BMP-2, une BMP-4 ou des hétérodimères de celles-ci.

16. Ecarteur selon la revendication 1, dans lequel lesdites extrémités de chacun desdits bras (120, 121) comportent une surface arquée configurée de sorte à recevoir une surface convexe d'un écarteur adjacent.

17. Ecarteur selon la revendication 1, dans lequel la région à emboîtement (170, 171) est tronquée en forme d'arc.

18. Ecarteur selon la revendication 1, en combinaison avec un deuxième écarteur adjacent, dans lequel ledit corps allongé (105) a un premier diamètre extérieur maximal, chacun desdits bras (120, 121) dudit corps allongé étant configuré de sorte à former une région à emboîtement pour l'emboîtement dudit deuxième écarteur adjacent, ayant un deuxième diamètre extérieur maximal, pour former un assemblage d'écarteur ayant une largeur inférieure à la somme des diamètres extérieurs maximaux combinés.

19. Ecarteur selon la revendication 8 composé d'os.

20. Ecarteur selon la revendication 1, dans lequel ledit corps allongé (105) comporte un axe longitudinal, ladite chambre (130) s'étendant perpendiculairement audit axe longitudinal.

21. Ecarteur de fusion intervertébrale selon la revendication 1, dans lequel:
ledit corps allongé (105) est composé d'os, et comporte un axe longitudinal et définit ladite chambre (130) s'étendant perpendiculairement audit axe longitudinal dudit corps, ledit corps d'os composé d'os prélevé de la diaphyse d'un os long comportant un canal médullaire, ladite région à emboîtement (170, 171) comportant une surface concave.

22. Ecatteur selon la revendication 21, dans lequel ledit corps comprend en outre une extrémité d'engagement d'un outil (101), définssent un trou d'engagement d'un outil (115) pour recevoir un outil d'enfoncement afin d'implanter l'écarteur.

23. Ecarteur de fusion intervertébrale selon la revendication 1, en combinaison avec:
un deuxième écarteur de fusion intervertébrale (100', 200) comportant un deuxième corps allongé (105', 204) composé d'os et une surface externe (206), ledit deuxième écarteur de fusion intervertébrale (100', 200) pouvant être emboîté dans ledit premier écarteur de fusion intervertébrale (100) dans lesdites régions à emboîtement (1.70, 171).

24. Ecarteur selon la revendication 23, dans lequel ledit deuxième corps allongé (204) définit un trou de passage (230).

25. Ecarteur selon la revendication 24, dans lequel ledit deuxième corps allongé (204) comporte un axe longitudinal, ledit trou de passage (230) s'étendant perpendiculairement audit axe longitudinal.

26. Ecarteur selon la revendication 23, dans lequel ledit deuxième corps allongé définit une deuxième chambre, ledit deuxième corps comportant un troisième bras et un quatrième bras opposé, lesdits troisième et quatrième bras définissant une deuxième ouverture en communication avec ladite deuxième chambre, ledit deuxième écarteur de fusion intervertébrale pouvant être emboîté dans ledit premier écarteur de fusion intervertébrale.

27. Ecarteur selon la revendication 26, dans lequel ledit système comprend en outre un matériau ostéogène disposé dans au moins une desdites chambres desdits écarteurs.

28. Combinaison d'écarteurs de fusion intervertébrale, comprenant:
un premier écarteur de fusion intervertébrale (100) selon l'une quelconque des revendications 1 à 22; et
un deuxième écarteur (100', 200) adjacent audit premier écarteur (100);
ledit deuxième écarteur (100', 200) ayant un deuxième diamètre extérieur maximal, ledit deuxième écarteur étant en relation à emboîtement avec ledit premier écarteur par une première partie emboîtée dans une région dudit premier bras (120) dudit premier écarteur et une deuxième partie emboîtée dans une région dudit deuxième bras (121) dudit premier écarteur;
ladite combinaison d'écarteurs de fusion intervertébrale ayant, par suite de ladite relation à emboîtement, une largeur globale inférieure à la somme dudit premier diamètre extérieur maximal et dudit deuxième diamètre extérieur maximal.
